# EUROPEAN PATENT APPLICATION

(11) **EP 0 937 709 A1**
(43) Date of publication of application: **25.08.1999**
(21) Application number: 99102289.8
(22) Date of filing: 05.02.1999
(51) Int. Cl.: C07C 311/08, A61K 31/63

(54) **Nimesulide micronised salts**

(30) Priority: 10.02.1998 IT MI980253
(71) Applicant: Helsinn Healthcare S.A., 6912 Pazzallo-Lugano (CH)
(72) Inventor: Monti, Tiziana, 6912 Pazzallo-Lugano (CH); Mossi, Waldo, 6710 Biasca (CH)
(74) Representative: Minoja, Fabrizio, Dr.

(57) **Abstract**

Nimesulide micronised salts with metals such as sodium, potassium, calcium, magnesium and zinc, have improved characteristics of bioavailability and pharmacokinetics.

## Description

The present invention relates to Nimesulide soluble salts.

Nimesulide, or 4-nitro-2-phenoxymethanesulfonanilide, is a non-steroidal antiinflammatory drug which, compared with other compounds of the same class, differs in its more marked therapeutical effects, in its very good tolerability and in the lower incidence of side effects, mainly on the gastrointestinal tract.

Nimesulide is sparingly soluble in water, thus involving problems in formulating some dosage forms, for example oral or injectable solutions, syrups, effervescent tablets, transdermal forms and the like.

In order to overcome such solubility problem, complexes with cyclodextrin (WO 91/17774 and WO 94/2177), L-lysine salt and its complex with cyclodextrins (WO 95/34533) and complex of nimesulide sodium salt with cyclodextrin (WO 94/28031) have been suggested.

Furthermore, nimesulide alkali and alkaline-earth salts have been disclosed in US 3840597, which also cites and in part describes salts with amines such as morpholine, methylcyclohexylamine, glucosamine, triethylamine.

The salification solves the problem of the solubility, which increases by two or more orders of magnitude.

Therefore, if, on the one hand, the problem can be solved through salification, on the other hand, as there is not a direct relationship between solubility and absorption in vivo, the problem of absorption rate of the nimesulide soluble form is still to be tackled. The rapidity of action is, of course, a highly desirable characteristic for anti-inflammatory and analgesic drugs and a higher solubility does not always corresponds to a more rapid or higher absorption.

It has now been found that the micronised forms of pharmaceutically acceptable nimesulide salts have surprising, favourable pharmacokinetics and bioavailability characteristics.

Micronisation can be carried out, according to the invention, by spray-drying or by grinding the precipitated salt in vibrational mills.

The optimum particle size of the salts for the preparation of solid formulations ranges from 5 to 50 µm, preferably from 5 to 20 µm.

The micronisation line comprises a feeding device, a doser (such as a screw feeder), a fluidized bed, a cyclone and a filter unit. The maximum pressure can reach 12 bars; the micronisation rate can be adapted to the requirements of the used salt.

The micronised salts of the invention comprise nimesulide salts with metals such as sodium, potassium, calcium, magnesium and zinc.

Pharmaceutically acceptable alkali and alkaline-earth salts are preferred, in particular the micronised sodium salt.

The preparation of the salts is carried out with conventional methods, adding for example an aqueous solution of alkali or alkaline-earth hydroxides, carbonates or bicarbonates to a suspension of nimesulide in water: the salt can previously be recovered in the non-micronised form by precipitation at low temperature or by freeze-drying, and subsequently be micronised according to the methods cited above. Alternatively, the salt can be obtained directly in the micronised form by spray-drying, suitably adapting the operative parameters.

The salts of the invention, in particular the micronised sodium salt, thanks to the increased adsorption rate and increased adsorbed amount of nimesulide, allow to reduce the amount of active ingredient to be administered, without reducing the therapeutical effect.

The invention also relates to pharmaceutical compositions containing the nimesulide micronised salts and suitable excipients and carriers.

The compositions of the invention will preferably contain the equivalent of 5--200 mg of acid nimesulide per unit dose. Suitable administration forms comprise drinkable solutions, syrups, granulates or effervescent tablets, sterile injectable solutions, transdermal lotions.

The following examples show the invention in greater detail.

### Example 1

### Preparation of Nimesulide sodium salt

A) 50 g of Nimesulide in 150 g of H₂O are added with 22.0 g of 30% NaOH, the resulting mixture is heated to 50-60°C to complete dissolution (orange colour). The resulting clear solution is cooled to 0°C, the precipitated product is filtered and washed with 50 ml H₂O at 0°C.
B) The product can be recovered as well using the spray-drying technique after preparation of the alkaline solution.
C) Analytical characteristics of the recovered Nimesulide sodium salt:
   ¹H-NMR (400 MHz, Solvent D₂0): 2.98 ppm (s, 3H, -NH-SO₂CH₃); 7.07 ppm (d, 2H, -C(2')-H)); 7.21 ppm (m, 1H, -C(4')-H)); 7.44 ppm (m, 3H, -C(3')-H), -C(6)-H)); 7.73 ppm (d, 1H, dm, -C(3)H); 7.99 ppm (d, 1H, do=9.0, -C(5)H).
   ¹³C-NMR (50MHz, solvent D₂0): 42.84 ppm (-NH-SO₂CH₃); 118.45 ppm (-C(3)); 121.36 ppm (-C(2') and -C(6')); 121.94 ppm (-C(4') or -C(5))); 124.62 ppm (-C(4') or -C(5))); 126.82 ppm (-C(6)); 133.31 ppm (-C(3') and C(5'))); 141.54 ppm (-C(NH-SO₂CH₃)); 150.82 ppm (-C(NO₂)); 151.40 ppm (-C(2)); 159.95 ppm (-C(1')).
   IR in agreement.
   HPLC purity: 99.9 %
   HPLC assay: 99.4 %

### Example 2

### Preparation of Nimesulide magnesium salt/[(nime)₂Mg](H₂O)ₓ

50 g of Nimesulide in 165 g of water are added with 4.81 g of Mg(OH)₂. The resulting reaction mixture is heated to 50-60°C, then filtered through Celite and crystallized at 0°C. The precipitated product is filtered and washed with 50 g of water at 0°C.

Spectroscopical characteristics:
¹H-NMR (400 MHz, Acetone-d₆) ppm: 2.96 (s, 3H, CH₃); 7.07 (d, 2H, C(2')-H, C(6')-H); 7.16 (m, 1H, C(4')-H); 7.41 (m, 2H, C(3')-H, C(5')-H); 7.59 (d, 2H, C(6)-H, C(5)-H); 7.95 (d, 1H, C(3)-H).
¹³C-NMR (50 MHz, Acetone-d₆) ppm: 40.68 (CH₃); 114.81 (C(3)); 119.18 (C(5), C(2'), C(6')); 121.38 (C(4')); 124.25 (C(6)); 130.69 (C(3'), C(5)); 138.78 (C(1)); 148.01 (C(2), C(4)); 157.69 (C(1')).

| Elementary analysis: Formula: [(nimesulide)₂Mg].2H₂O | | | |
|---|---|---|---|
| Calculated: | C: 46.26%; | H: 3.88%; | N: 8.30%. |
| Found: | C: 46.15%; | H: 3.84%; | N: 8.26%. |

The sample is hygroscopic. Water content increases after exposure to air (3 months).

### Example 3

### Preparation of Nimesulide calcium salt/[(nime)₂Ca](H₂O)ₓ

50 g of Nimesulide in 165 g of water are added with 6.11 g of Ca(OH)₂, the resulting mixture is heated to 50-60°C to complete dissolution (orange colour). The resulting clear solution is cooled to 0°C, the precipitated product is filtered and washed with 50 g of water.

Spectroscopical characteristics:
¹H-NMR (400 MHz, D₂0) ppm: 2.92 (s, 3H, CH₃); 7.00 (d, 2H, C(2')-H, C(6')-H); 7.14 (m, 1H, C(4')-H); 7.37 (m, 3H, C(3')-H, C(5')-H, C(6)-H); 7.64 (d, 1H, C(3)-H); 7.93 (d, 1H, C(5)-H).
¹³C-NMR (50 MHz, D₂O) ppm: 42.25 (CH₃); 117.84 (C(3)); 120.76 (C(2'), C(6')); 121.38 (C(5)); 124.04 (C(4')); 126.21 (C(6)); 132.70 (C(3'), C(5')); 140.92 (C(1)); 150.27 (C(4)); 150.96 (C(2)); 159.40 (C(1')).

| Elementary analysis: Formula: [(nimesulide)₂Ca].2H₂O | | | |
|---|---|---|---|
| Calculated: | C: 45.20%; | H: 3.79%; | N: 8.11%. |
| Found: | C: 45.23%; | H: 3.95%; | H: 8.16%. |

After three months exposure to air, the sample turns out to be hygroscopic:

| Formula: [(nimesulide)₂Ca].3H₂O | | | |
|---|---|---|---|
| Calculated: | C: 44.06%; | H: 3.98%; | N: 7.91%. |
| Found: | C: 44.11%; | H: 4.21%; | N: 7.77%. |

Water content (according to Karl Fisher):
Formula: [(nimesulide)₂Ca].3H₂O
Calculated: 7.63%.
Measured: 7.82%.

### Example 4

### Preparation of Nimesulide potassium salt/[(nime)K](H₂O)ₓ

50 g of Nimesulide in 165 g of water are added with 10.9 g of 85% KOH, the resulting mixture is heated to 50-60°C to complete dissolution (orange colour). The resulting clear solution is cooled to 0°C, the precipitated product is filtered and washed with 50 g of water.

Spectroscopical characteristics:
¹H-NMR (400 MHz, D₂O) ppm: 2.90 (s, 3H, CH₃); 6.94 (d, 2H, C(2')-H, C(6')-H); 7.11 (m, 1H, C(4')-H); 7.33 (m, 3H, C(3')-H, C(5')-H, C(6)-H); 7.51(d, 1H, C(3)-H); 7.84 (d, 1H, C(5)-H).
¹³C-NMR (50 MHz, D₂O) ppm: 42.14 (CH₃); 117.65 (C(3)); 120.83 (C(2'), C(6')); 121.20 (C(5)); 124.06 (C(4')); 126.28 (C(6)); 132.73 (C(3'), C(5')); 140.72 (C(1)); 150.30 (C(4)); 151.15 (C(2)); 159.33 (C(1')).

| Elementary analysis: Formula: [(nimesulide)K].1/3H₂O | | | |
|---|---|---|---|
| Calculated: | C: 44.31%; | H: 3.34%; | N: 7.95%. |
| Found: | C: 44.39%; | H: 3.43%; | N: 7.91%. |

## Claims

1. Nimesulide pharmaceutically acceptable salts in the micronised form.

2. Salts as claimed in claim 1 selected from sodium, potassium, calcium, magnesium and zinc salts.

3. Nimesulide micronised sodium salt.

4. Pharmaceutical forms containing a salt of claims 1-3 in admixture with suitable carriers or excipients.
